# EUROPEAN PATENT APPLICATION

(11) **EP 1 944 035 A1**
(43) Date of publication of application: **16.07.2008**
(21) Application number: 08006029.6
(22) Date of filing: 07.05.2003
(51) Int. Cl.: A61K 35/76, A61P 25/04, A61P 29/00, A61P 35/00

(54) **Method for reducing pain using oncolytic viruses**

(30) Priority: 09.05.2002 US 378675 P; 29.01.2003 US 443177 P
(62) Divisional of application: 03722131.4
(71) Applicant: Oncolytics Biotech Inc., Calgary, AB T2N 1X7 (CA)
(72) Inventor: Morris, Donald, Clagary, Alberta T3R 1B1 (CA); Coffey, Matthew c., Calgary, Alberta T2N 3L4 (CA); Thompson, Bradley G., Calgary, Alberta T2N 0Z5 (CA)
(74) Representative: Vossius & Partner

(57) **Abstract**

The present invention provides a method for reducing pain associated with neoplasms in a mammal, comprising administering an effective amount of one or more oncolytic viruses. Preferably, the mammal also receives an analgesic, and the amount of analgesic required by the mammal is reduced when the oncolytic virus is administered. The oncolytic virus is preferably reovirus. The mammal may be additionally subject to chemotherapy, immunotherapy, hormonal and/or radiation therapy.

## Description

### RELATED APPLICATIONS

This application claims the benefit of U.S. Provisional Applications Serial Number 60/378,675, filed May 9, 2002; and Serial Number 60/443,177, filed January 29, 2003. The entire disclosure of these prior applications is hereby incorporated by reference.

### FIELD OF THE INVENTION

The present invention pertains to methods for reducing pain associated with proliferative disorders in a mammal using oncolytic viruses, particularly reovirus.

### REFERENCES

U.S. Patent 5,023,252.
U.S. Patent 6,110,461.
U.S. Patent 6,136,307.
U.S. Patent 6,261,555.
U.S. Patent 6,344,195.
U.S. Patent Application Publication No. 20020037576.
WO 94/18992, published September 1, 1994.
Bar-Eli, N., et al., "preferential cytotoxic effect of Newcastle disease virus on lymphoma cells", J. Cancer Res. Clin. Oncol. 122: 409-415 (1996).
Blagoslelonny, M.V., et al., "in vitro Evaluation of a p53-Expressin g Adenovirus as an Anti-Cancer Drug", Int. J. Cancer 67(3):386-392 (1996).
Chandron and Nibert, "Protease cleavage of reovirus capsid protein mu1 and mu1C is blocked by alkyl sulfate detergents, yielding a new type of infectious subvirion particle" J. of Virology 72(1):467-75 (1998).
Chang et al., PNAS 89:4825-4829 (1992).
Chang, H.W. et al., Virology 194:537-547 (1993).
Chang et al., J. Virol. 69:6605-6608 (1995).
Cleary J. "Cancer Pain Management" Cancer Control 7: 120-131 (2000).
Cleeland C., et a1. "Pain and its treatment in outpatients with metastatic cancer" N Engl J Med. 330: 592-596 (1994).
Cuff et al., "Enteric reovirus infection as a probe to study immunotoxicity of the gastrointestinal tract" Toxicological Sciences 42(2):99-108 (1998).
Duncan et al., "Conformational and functional analysis of the C-terminal globular head of the reovirus cell attachment protein" Virology 182(2):810-9 (1991).
Fields, B.N. (ed.) Fundamental Virology (3rd Edition), Lippencott-Raven Press (1996).
Fueyo, J., et al., "A Mutant Oncolytic Adenovirus Targeting the Rb Pathway Produces Anti-Glioma Effect in Vivo", Oncogene 19(1):2-12 (2000).
Harlow, E., et al., "Antibodies. A Laboratory Manual". CSH Laboratories, New York, 1988.
Kawagishi-Kobayashi, M., et al., Mol. Cell. Biology 17:4146-4158 (1997).
Lesage P. and Portenoy R.K. "Trends in Cancer Pain Management" Cancer Control 6: 136-145 (1999).
Mah et al., "The N-terminal quarter of reovirus cell attachment protein sigma 1 possesses intrinsic virion-anchoring function" Virology 179(1):95-103 (1990).
The Merck Manual (17th ed.), particularly Chapter 167. Merck Research Laboratories, New Jersey (1999).
Merskey H, Bogduk N, eds. "Classification of Chronic Pain: Descriptions of Chronic Pain Syndromes and Definitions of Pain Terms," Prepared by the Task Force on Taxonomy of the International Association for the Study of Pain. 2nd ed. Seattle, Wash: IASP Press (1994).
Miguel R. "Interventional Treatment of Cancer Pain: The Fourth Step in the World Health Organization Analgesic Ladder?" Cancer Control, 7:149-156 (2000).
Nemunaitis, J., Invest. New Drugs 17:375-386 (1999).
Nibert, et al. "Reoviruses and their replication", pages 1557-96 in Fundamental Virology (Fields et al., 3rd Edition), Lippencott-Raven Press (1996).
Portenoy R., et al. "Symptom prevalence, characteristics and distress in a cancer population" Qual Life Res. 3: 183-189 (1994).
Reichard, K.W., et al., "Newcastle Disease Virus Selectively Kills Human Tumor Cells", J. of Surgical Research 52:448-453 (1992).
Remington's Pharmaceutical Sciences, 18th Ed., Mack Publishing Company, Pennsylvania, 1990.
Romano et al., Mol. and Cell. Bio. 18:7304-7316 (1998).
Sharp et al., Virol. 250:301-315 (1998).
Turner and Duncan, "Site directed mutagenesis of the C-terminal portion of reovirus protein sigma1: evidence for a conformation-dependent receptor binding domain" Virology 186(1):219-27 (1992).
World Health Organization. "Cancer Pain Relief: With a Guide to Opioid Availability", *Geneva, Switzerland: WHO* (1996).
Yoon, S.S., et al., "An Oncolytic Herpes Simplex Virus Type I Selectively Destroys Diffuse Liver Metastases from Colon Carcinoma", FASEB J.. 14:301-311(2000).
Zorn, U. et al., "Induction of Cytokines and Cytotoxicity against Tumor Cells by Newcastle Disease Virus", Cancer Biotherapy 9(3):22-235 (1994).

All of the publications, patents and patent applications cited above or elsewhere in this application are herein incorporated by reference in their entirety to the same extent as if the disclosure of each individual publication, patent application or patent was specifically and individually indicated to be incorporated by reference in its entirety.

### BACKGROUND OF THE INVENTION

Pain is a prevalent symptom in cancer patients, affecting up to 50 % of patients undergoing active cancer treatment and up to 90 % of those with advanced disease. (Portenoy, et al., 1994; Cleeland, et al., 1994). The World Health Organization, international and national professional organizations, and governmental agencies of the United States and other countries have all acknowledged the importance of pain management as part of routine cancer care (Miguel, 2000).

Current methods of treatment of pain in cancer patients are primarily based on drug therapy including opioid therapy, non-opioid and adjuvant analgesics. However, narcotics such as morphine have the disadvantages of addiction and development of tolerance. The use of non-opioid and adjuvant analgesics is limited by side effects and concerns about gastrointestinal and renal toxicity (Cleary, 2000).

For patients who do not respond adequately to drug therapy, alternative analgesic therapies are considered: These therapies include anesthesiologic, surgical, and neurostimulatory interventions. For example, destructive neurolysis using phenol or alcohol is considered when other nondestructive approaches are not possible or have failed, the pain is well localized, and the block will not compromise strength or sphincter function. Although some neurostimulatory techniques can be relatively noninvasive (for example, transcutaneous electrical nerve stimulation and acupuncture), others are still invasive (for example, dorsal column stimulation or deep brain stimulation). Neurosurgical techniques directed against specific peripheral or central nervous system structures can only benefit a highly selected group with refractory cancer related pain. Cordotomy is most often used (Lesage and Portenoy, 1999).

In view of the drawbacks associated with the current means for treating pain in cancer patients, the need still exists for improved methods for pain treatment which do not have negative side effects or toxicity, are not invasive and are not associated with the development of tolerance.

### SUMMARY OF THE INVENTION

The present invention relates to the surprising discovery that oncolytic viruses, in particular reovirus, can reduce pain associated with proliferative disorders, particularly with the growth of a solid tumor mass. Reovirus is a highly selective oncolytic antitumor agent, which replicates selectively in ras-activated cells with the subsequent lytic exit from the cells (see, e.g., U.S. Patents Nos. 6,110,461; 6,136,307; 6,261,555; and 6,344,195). In the present invention, we demonstrate that virus administration resulted in diminished symptoms of direct tumor-associated pain. This lessening of pain occurred both with and without actual tumor regression. Furthermore, there were no significant side effects or adverse effects related to the administration of reovirus.

Accordingly, the present invention provides a method for reducing pain associated with a neoplasm in a mammal comprising administering to the mammal an effective amount of one or more oncolytic viruses. Preferably, the mammal suffers from pain due to the neoplasm, and relies on analgesic measures to reduce pain prior to being treated with oncolytic viruses. With oncolytic viruses, however, the amount of analgesic measures required by the mammal to reduce pain is lower than that in the absence of oncolytic viruses. The analgesic measure may be an analgesic drug or a non-drug measure, such as surgery. The analgesic drug may be selected from the group consisting of opioid analgesics, non-opioid analgesics, anesthetics and alpha-2 adrenergic agonists.

Preferably, the oncolytic virus is reovirus. The reovirus may be a mammalian reovirus or an avian reovirus. Preferably, the reovirus is a human reovirus. The human reovirus may be any of the three serotypes: type 1 (strain Lang or T1L), type 2 (strain Jones, T2J) and type 3 (strain Dearing or strain Abney, T3D). Preferably, the human reovirus is serotype 3 reovirus. The reovirus may be modified such that the outer capsid is removed, the virion is packaged in a liposome or micelle, or the proteins of the outer capsid have been mutated. The reovirus can be administered in a single dose or in multiple doses.

The reovirus may also be a recombinant reovirus. The recombinant reovirus may be generated by co-infection of mammalian cells with different subtypes of reovirus. The recombinant reovirus may be naturally-occurring or non-naturally-occurring. The recombinant reovirus may be from two or more strains of reovirus, particularly two or more strains of reovirus selected from the group consisting of strain Dearing, strain Abney, strain Jones, and strain Lang. The recombinant reovirus may also result from reassortment of reoviruses from different serotypes, such as selected from the group consisting of serotype 1 reovirus, serotype 2 reovirus and serotype 3 reovirus. The recombinant reovirus may comprise naturally-occurring variant coat protein coding sequences or mutated coat protein coding sequences.

More than one kind of oncolytic viruses may be administered to the mammal. For the same kind of oncolytic viruses, more than one type or strain may be administered.

The neoplasm may be a solid neoplasm. The neoplasm may be metastatic and/or terminal. The virus may be administered by injection into or near a solid neoplasm, or by any other methods known in the art, such as systemic administration.

Preferably, the mammal is selected from dogs, cats, rodents, sheep, goats, cattle, horses, pigs, humans and non-human primates. More preferably, the mammal is a human. The mammal may be subject to chemotherapy, immunotherapy, hormonal or radiation therapy in conjunction with the oncolytic viruses.

Also provided is a pharmaceutical composition for reducing pain in a mammal, comprising an oncolytic virus and an analgesic. The composition may also comprise a pharmaceutically acceptable excipient. The virus may be a modified virus. Preferably, the oncolytic virus is a reovirus. The reovirus may also be a recombinant reovirus. The recombinant reovirus may be generated by co-infection of mammalian cells with different subtypes of reovirus. The recombinant reovirus may be naturally-occurring or non-naturally-occurring. The recombinant reovirus may be from two or more strains of reovirus, particularly two or more strains of reovirus selected from the group consisting of strain Dearing, strain Abney, strain Jones, and strain Lang. The recombinant reovirus may also result from reassortment of reoviruses from different serotypes, such as selected from the group consisting of serotype 1 reovirus, serotype 2 reovirus and serotype 3 reovirus. The recombinant reovirus may comprise naturally-occurring variant coat protein coding sequences or mutated coat protein coding sequences.

Further provided is a pharmaceutical composition for reducing pain in a mammal suffering from a neoplasm, comprising two components as follows:
(a) a first component which is a first pharmaceutical composition comprising an effective amount of an oncolytic virus; and
(b) a second component which is a second pharmaceutical composition comprising an effective amount of an analgesic.
Preferably, the amount of the analgesic is less than that required if the first component is absent.

Each component may also comprise a pharmaceutically acceptable excipient. Furthermore, the two components may be administered separately. The analgesic is preferably selected from the group consisting of opioid analgesics and non-opioid analgesics. In particular, the oncolytic virus may be a reovirus. The reovirus may also be a recombinant reovirus.

Another aspect of the present invention provides a kit that comprises an oncolytic virus and an analgesic. The kit may further comprise any component that can be used to practice the present invention as disclosed herein. For example, the kit may further comprise at least one additional oncolytic virus, a chemotherapeutic agent, an immunosuppresive agent, a means for suppressing the immune system, an anti-antivirus antibody, a means for administering any of the components of the kit, and/or an instruction for using the kit.

The methods; pharmaceutical compositions and kits of the invention provide an effective means for reducing pain associated with proliferative disorders in a mammal, without the side effects associated with other forms of pain treatment. In the case of reovirus, because reovirus is not known to be associated with disease, any safety concerns associated with deliberate administration of a virus are minimized.

The foregoing and other objects, features and advantages of the invention will be apparent from the following more particular description of preferred embodiments of the invention.

### BRIEF DESCRIPTION OF THE DRAWINGS

**Figure 1**

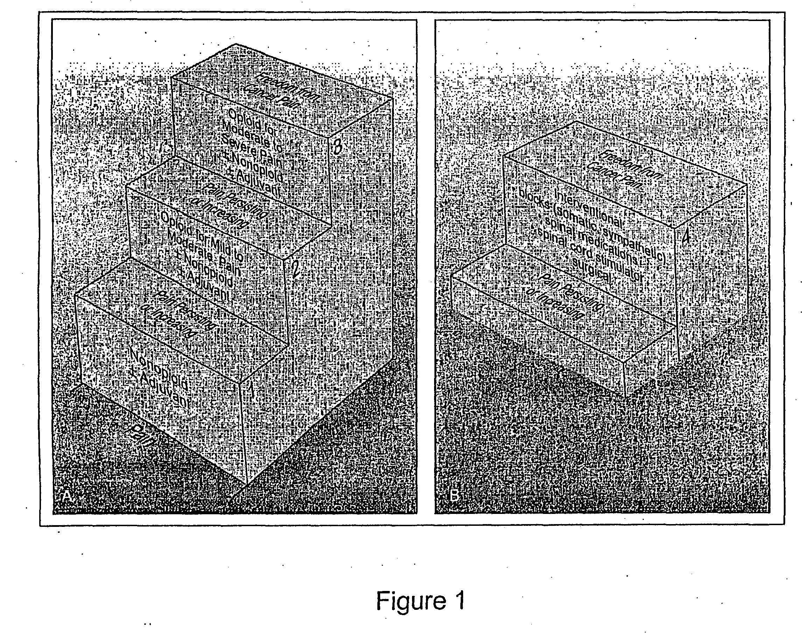

   Figure 1A shows the 3-step analgesic ladder developed by the World Health Organization. Figure 1B shows the 4^{th} step proposed in Miguel, 2000.

### DETAILED DESCRIPTION OF THE INVENTION

The present invention relates to the surprising discovery that oncolytic viruses, in particular reovirus, can reduce pain associated with proliferative disorders, particularly with the growth of a solid tumor mass. Reovirus is a highly selective oncolytic antitumor agent, which replicates only in ras-activated cells with the subsequent lytic exit from the cells. In the present invention, we demonstrate that virus administration resulted in diminished symptoms of direct tumor-associated pain. This lessening of pain occurred both with and without actual tumor regression. Furthermore, there were no significant side effects or adverse effects related to the administration of the reovirus.

Prior to describing the invention in further detail, the terms used in this application are defined as follows unless otherwise indicated.

### Definitions

"Pain, " as used herein, refers to an unpleasant sensory and emotional experience which is primarily associated with tissue damage or described in terms of such damage, or both (Merskey et al, eds., 1994).

"Reducing pain, " as used herein, refers to alleviating pain in a mammal in order to improve the well-being and thus quality of life of the mammal. A pain is reduced when, for example, a lower amount of a given analgesic is required, it requires a lesser step in the analgesic ladder (see below), or it requires less frequent use of analgesics. The reduction in the amount or frequency of analgesics is preferably at least about 10%, more preferably at least about 20 %, 30 %, 40 %, 50%, 60 %, 70 %, 80 % or 90 %, and most preferably 100% (i.e., analgesic is no longer needed). The reduction in analgesic steps may be one step; for example, a patient usually needs the Step 4 treatment for pain but it is reduced to Step 3. The reduction in analgesic steps is preferably two or three steps and most preferably four steps.

A "tumor pain" or "cancer pain" is a pain associated with a proliferative disorder. The pain may be directly due to the presence of the disorder, or indirectly associated with the disorder, such as resulting from an operation performed for the disorder.

The term "nociceptive" is applied to pains that are presumed to be maintained by ongoing tissue injury. Nociceptive pain is called somatic when the ongoing activation is related to primary afferent nerves in somatic tissues (for example, bone, joint, or muscle) and visceral when viscera afferents are activated by injury. In general, visceral pain is described as a diffuse or pressure-type sensation, which is poorly localized. Somatic pain has a squeezing sharp pain nature that the patient can locate exactly. Pains that are nociceptive, such as bone pain, are the most prevalent type associated with cancer.

"Neuropathic pain" is pain that is believed to be sustained by aberrant somatosensory processing in the peripheral or central nervous system. Neuropathic pain may present as a burning, tingling sensation with a lancinating component. It can be further subdivided into deafferentation pains (such as central pain, phantom pain, and postherpetic neuralgia), peripheral mononeuropathies and polyneuropathies, and the complex regional pain syndromes (reflex sympathetic dystrophy or causalgia). Neuropathic pain syndromes respond less well to opioid drugs than nociceptive pain syndromes.

Pain syndromes can be "acute" or "chronic."

"Acute pain" syndromes are usually caused by common diagnostic or therapeutic interventions. Acute pain may be also due to the neoplasm (for example, vertebral collapse and other pathological fractures, acute obstruction of hollow viscus, and hemorrhage into tumor) or related pathology (for example, acute pain associated with infection: myalgia and arthralgia associated with sepsis, pain associated with superficial wounds or abscesses).

Acute pain may be associated with diagnostic procedures including, for example, lumbar puncture, bone marrow biopsy, and paracentesis. Acute pain may be associated with therapeutic procedures including, for example, pleurodesis, tumor embolization, and nephrostomy insertion. Acute pain may be associated with analgesic procedures including, for example, spinal opioid hyperalgesia syndrome, and pain following strontium-89 therapy. Acute pain may be also associated with therapies including chemotherapy (for example, intraperitoneal chemotherapy, oropharyngeal mucositis, and peripheral neuropathy), hormonal therapy (for example, painful gynecomastia, and hormone-induced acute pain flare), immunotherapy (for example, arthralgia and myalgia from interferon and interleukin), and radiation therapy (for example, oropharyngeal mucositis, acute radiation enteritis and proctolitis, and brachial plexopathy).

Acute flares of pain also are highly prevalent among those with chronic pain. One half to two thirds of patients with well-controlled chronic pain experience transitory "breakthrough" pains (World Health Organization, 1996).

"Chronic pain" syndromes result primarily from a direct effect of the neoplasm. They also may be therapy-related or represent disorders unrelated to the disease or its treatment.

An "analgesic drug" is a substance that can reduce pain in a mammal. An "analgesic" is an analgesic drug or other measure (such as surgery) used to reduce pain in a mammal. Analgesic drugs include opioid analgesics and non-opioid analgesics.

"Opioid analgesics", "opioid", or "narcotic analgesics" are natural or synthetic substances that bind to opioid receptors in the central nervous system, producing an agonist action. Opioid analgesics are known in the art (see, *e*.*g*., The Merck Manual, 1999). Examples of opioid analgesics include, but are not limited to, codeine, fentanyl, hydromorphone, levorphanol, meperidine, methadone, morphine, oxycodone, oxymorphone, propoxyphene, buprenorphine, butorphanol, dezocine, nalbuphine, and pentazocine.

"Non-opioid analgesics" are natural or synthetic substances that reduce pain but are not opioid analgesics. Non-opioid analgesics are known in the art (see, e.g., The Merck Manual, 1999). Examples of non-opioid analgesics include, but are not limited to, etodolac, indomethacin, sulindac, tolmetin, nabumetone, piroxicam, acetaminophen, fenoprofen, flurbiprofen, ibuprofen, ketoprofen, naproxen, naproxen sodium, oxaprozin, aspirin, choline magnesium trisalicylate, diflunisal, meclofenamic acid, mefenamic acid, and phenylbutazone.

A "neoplastic cell", "tumor cell", or "cell with a proliferative disorder", refers to a cell which proliferates at an abnormally high rate. A new growth comprising neoplastic cells is a "neoplasm," also known as a "tumor." A tumor is an abnormal tissue growth, generally forming a distinct mass, that grows by cellular proliferation more rapidly than normal tissue growth. A tumor may show partial or total lack of structural organization and functional coordination with normal tissue. As used herein, a tumor is intended to encompass hematopoietic tumors as well as solid tumors.

A tumor may be benign (benign tumor) or malignant (malignant tumor or cancer). Malignant tumors can be broadly classified into three major types. Malignant tumors arising from epithelial structures are called carcinomas, malignant tumors that originate from connective tissues such as muscle, cartilage, fat or bone are called sarcomas and malignant tumors affecting hematopoietic structures (structures pertaining to the formation of blood cells) including components of the immune system, are called leukemias and lymphomas. Other tumors include, but are not limited to neurofibromatosis.

A "proliferative disorder", also referred to as a "neoplasm" or "tumor", is a disease or condition caused by cells which grow more quickly than normal cells, i.e., neoplastic cells. Proliferative disorders include benign tumors and malignant tumors. When classified by structure of the tumor, proliferative disorders include solid tumors and hematopoietic tumors.

"Ras-activated neoplastic cells" or "ras-mediated neoplastic cells" refer to cells which proliferate at an abnormally high rate due to, at least in part, activation of the ras pathway. The ras pathway may be activated by way of ras gene structural mutation, elevated level of ras gene expression, elevated stability of the ras gene message, or any mutation or other mechanism which leads to the activation of ras or a factor or factors downstream or upstream from ras in the ras pathway, thereby increasing the ras pathway activity. For example, activation of EGF receptor, PDGF receptor or sos results in activation of the ras pathway. Ras-mediated neoplastic cells include, but are not limited to, ras-mediated cancer cells, which are cells proliferating in a malignant manner due to activation of the ras pathway.

A "terminal cancer", as used herein, refers to a cancer that is inoperable and where a treatment is no longer possible.

A "metastatic tumor" is a tumor that has metastasized from a tumor located at another place in the same animal.

"Oncolytic viruses" are viruses which are capable of destruction of tumor cells. Generally, oncolytic viruses are replication-competent viruses with experimentally determined replicative advantages in tumor cells compared with normal tissue. An ideal oncolytic virus introduced by a single intratumor inoculation replicates and lyses the infected cell, and subsequently infects the surrounding tumor cells until it reaches the boundary between malignant and normal tissue. Oncolytic viruses include, for example, adenovirus derivatives, HSV-1 derivatives, autonomous parvovirus, poxvirus, Newcastle disease virus, poliovirus derivatives, vesicular stomatitis virus, influenza virus derivatives and reovirus.

The virus may be naturally occurring or modified. The virus is "naturally-occurring": when it can be isolated from a source in nature and has not been intentionally modified by humans in the laboratory. For example, the virus can be from a "field source", that is, from an infected animal.

The virus may be chemically or biochemically pretreated (e.g., by treatment with a protease, such as chymotrypsin or trypsin) prior to administration to the recipient. Pretreatment with a protease removes the outer coat or capsid of the virus and may increase the infectivity of the virus. The virus may be coated in a liposome or micelle (Chandron and Nibert, (1998) to reduce or prevent an immune response from a mammal which has developed immunity to the virus. For example, the virion may be treated with chymotrypsin in the presence of micelle forming concentrations of alkyl sulfate detergents to generate a new infectious subvirion particle.

An "immunoprotected virus" is a virus modified to reduce or eliminate an immune reaction to the virus.

"Infection by virus" refers to the entry and replication of virus in a cell. Similarly, "infection of a tumor by virus" refers to the entry and replication of virus in the cells of the tumor.

"Reovirus" refers to any virus classified in the reovirus genus, whether naturally occurring, modified or recombinant. Reoviruses are viruses with a double-stranded, segmented RNA genome. The virions measure 60-80 nm in diameter and possess two concentric capsid shells, each of which is icosahedral. The genome consists of double-stranded RNA in 10-12 discrete segments with a total genome size of 16-27 kbp. The individual RNA segments vary in size. Three distinct but related types of reovirus have been recovered from many species. All three types share a common complement-fixing antigen.

The human reovirus consists of three serotypes: type 1 (strain Lang or T1L), type 2 (strain Jones, T2J) and type 3 (strain Dearing or strain Abney, T3D). The three serotypes are easily identifiable on the basis of neutralization and hemagglutinin-inhibition assays (see, for example, Fields, B.N. *et al*., 1996).

An "effective amount" is an amount of a substance which is sufficient to result in the intended effect. For virus used to reduce pain, an efficient amount is an amount of virus sufficient to alleviate or eliminate pain.

"Administration to a neoplastic cell or neoplasm" indicates that the virus is administered in a manner so that it contacts the neoplastic cells or cells of the neoplasm (i.e., neoplastic cells).

The term "unit dosage forms" refers to physically discrete units suitable as unitary dosages for human subjects and other mammals, each unit containing a predetermined quantity of virus calculated to produce the desired effect, in association with a suitable pharmaceutical excipient.

### Tumor Pains

The incidence and severity of pain vary depending on the type of tumor. For example, Table 1 lists several cancer types and their association with pain (Miguel, 2000).

**Table 1**

| **Cancer Type and Its Association with Pain** | |
|---|---|
| **Type of Cancer** | **Patients with Pain (%)** |
| Bone | 85 |
| Oral Cavity | 80 |
| Genitourinary (men/women) | 75/78 |
| Breast | 52 |
| Lung | 45 |
| Gastrointestinal | 40 |
| Lymphoma | 20 |
| Leukemia | 5 |

Pain may be nociceptive (somatic or visceral) or neuropathic. Tumor-related nociceptive pain syndromes can be due to neoplastic invasion of bone, joint, muscle or connective tissue. Bone pain syndromes are the most prevalent. Bone metastases are often painless, and the factors that distinguish a painful lesion from a painless one are poorly understood. Multifocal bone pain is usually caused by widespread metastases.

The spine is the most common site of bone metastases, and back pain is an extremely common problem in the cancer population. Any neoplastic lesion of the vertebra has the potential to damage spinal cord or nerve roots and produce devastating neurological compromise. Specific pain patterns (for example, "crescendo" pain, pain flare with recumbency, or radicular pain), specific neurological findings (for example, radiculopathy), and specific radiological findings (for example, 50% collapse of a vertebral body) are suspicious of epidural compression. Epidural spinal cord compression is a compelling example of the value of syndrome recognition in cancer pain assessment.

Visceral nociceptive pain syndromes can result from obstruction, infiltration, or compression of visceral structures, including hollow, viscous and supporting connective tissues.

Tumor-related neuropathic pain syndromes may be caused by tumor infiltration or compression of nerve, plexus, or roots, or by the remote effects of malignancy on peripheral nerves. These syndromes are highly variable. The character of the pain can be aching or dysesthetic (abnormal pain sensations, such as burning), the location can be anywhere in the dermatomal region innervated by the damaged neural structure, and the dysfunction may or may not be motor, sensory, or autonomic.

Treatment-related pain syndromes may be related to antineoplastic therapies. Nociceptive pains related to chemotherapy, radiation therapy, or surgery appear to be uncommon. Radiation or corticosteroid-based chemotherapy regimens can induce osteonecrosis of bones, and chronic visceral pain can follow intraperitoneal chemotherapy or abdominal radiation therapy. These syndromes can simulate tumor-related pains, and the exclusion of recurrence constitutes a major challenge.

Most posttreatment pain syndromes are neuropathic. The predisposing factors for chronic neuropathic pain following nerve injury are unknown. Any surgical incision, even minor, can induce a neuropathic pain syndrome. For example, the postmastectomy syndrome, which may be precipitated by injury to the intercostobrachial nerve, causes a tight, burning sensation in the medial aspect of the upper arm, the axilla, and the upper aspect of the anterior chest wall. This pain is not associated with tumor recurrence. In contrast, persistent or recurrent pain after thoracotomy can be treatment related, but it is usually related to the neoplasm.

Radiation-induced fibrosis can cause peripheral nerve injury. The resultant chronic neuropathic pain usually appears months to years following treatment. Contrary to nerve injury related to neoplasm, the pain is generally less prominent and slowly progressive. It is often associated with weakness, sensory disturbances, radiation changes of the skin, and lymphedema.

Painful dysesthesias, paresthesias, cramps, and restless legs associated with mild weakness, sensory loss, or autonomic dysfunction may follow treatment with neurotoxic chemotherapy (for example, vincristine, cisplatin, paclitaxel). Although most patients report gradual improvement after therapy is discontinued, some develop a persistent, painful polyneuropathy.

### The Analgesic Ladder

In 1986, the World Organization established a three-step ladder as a guideline for the treatment of cancer pain (Figure 1A). Thus, non-opioid is used in step 1 with or without adjuvant analgesic. If the pain persists or increases, opioid for mild to moderate pain is used in step 2, with or without non-opioid/adjuvant analgesic. If the pain still persists or increases, opioid for moderate to severe pain is used in step 3, with or without non-opioid/adjuvant analgesic.

These three steps are more effective for somatic or visceral pains than for neuropathic pains. Therefore, a fourth step was proposed for the cases in which pain persists or increases after step 3, mainly with neuropathic pains (Miguel, 2000). In step 4, interventional measures are employed, such as surgery (for example, to inactivate nerves), spinal medications (epidural or subarachnoid) and nerve blockade (Figure 1B). Thus, neuropathic pain may be treated with the following analgesic measures:
(1) Neuroactive oral medicines, including tricyclic antidepressants (such as amitriptyline and notriptyline), anticonvulsants (such as gabapentin, DPH and carbamazepine), and oral local anesthetics (such as mexlitine);
(2) Nerve blockade, including the use of local anesthetics near nerves that transmit information relating to pain, or neurolytic agents to destroy nerves and interrupt pain pathways (neurolysis). Neurolysis may be chemical, thermic or surgical. At the present, chemical neurolysis is generally limited to alcohol or phenol;
(3) Spinal medications, including subarachnoid and/or epidural administration of drugs. This measure can be used for visceral and somatic pain as well. The drugs that can be administered in this manner include:

| *For visceral and somatic pain* | |
|---|---|
| opioids: | morphine, hydromorphone, fentanyl, sufentanil |
| local anesthetics: | lidocaine, bupivacaine, tetracaine |
| *For neuropathic pain* | |
| local anesthetics: | lidocaine, bupivacaine, tetracaine |
| alpha-2 adrenergic agonists: | clonidine, dexmedetomidine, guanabenz |
| antispasmodics: | baclofen; |

(4) Spinal cord stimulation, which involves using electricity to reduce pain. In general, patients control the stimulation (on/off and intensity) with a small battery-operated control.

### Methods

The present invention provides a method for reducing cancer pains using oncolytic viruses. Preferably, the pain is reduced to the extent that a lower amount or frequency of analgesic measures is required, or a lesser step in the analgesic ladder is necessary, as compared to the amount, frequency or step required in the absence of the viruses.

Based on our recent findings that intratumoral injection of reovirus is capable of causing tumor regression in a variety of animal models in Ras activated cells, a Phase 1 clinical study was conducted to examine any oncolytic effect of the virus in human patients with metastatic cancer that had failed to respond to conventional therapies. The trial established that the virus promoted tumor regression in a variety of solid tumor indications when administered intratumorally. Surprisingly, it was also noted that virus administration can result in diminished symptoms of direct tumor associated pain in this patient population. This lessening of pain has occurred with or without actual tumor regression. In one instance the reduction of pain was sufficient to permit the patient to discontinue narcotic intake. Further, there were no reports of significant side effects or adverse effects related to the administration of the virus in these patients.

Based upon these discoveries, Applicants have developed methods for treating pain associated with proliferative disorders in mammals by administration of oncolytic virus. Representative mammals include dogs, cats, sheep, goats, cattle, horses, pigs, non-human primates, and humans. In a preferred embodiment, the mammal is a human. In a preferred embodiment, the pain is associated with the growth of a solid tumor mass.

In the methods of the invention, virus is administered to an individual mammal. In a preferred embodiment, reovirus is used. Representative types of human reovirus that can be used include type 1 (e.g., strain Lang or T1L); type 2 (e.g., strain Jones or T2J); and type 3 (e.g., strain Dearing or strain Abney, T3D or T3A); other strains of reovirus can also be used. In a preferred embodiment, the reovirus is human reovirus serotype 3, more preferably the reovirus is human reovirus serotype 3, strain Dearing. Alternatively, the reovirus can be a non-human mammalian reovirus (e.g., non-human primate reovirus, such as baboon reovirus; equine; or canine reovirus), or a non-mammalian reovirus (e.g., avian reovirus). A combination of different serotypes and/or different strains of reovirus, such as reovirus from different species of animal, can be used.

The reovirus may be a recombinant reovirus resulting from the recombination/reassortment of genomic segments from two or more genetically distinct reoviruses. The recombinant reovirus may be from two or more types of reoviruses with differing pathogenic phenotypes such that it contains different antigenic determinants, thereby reducing or preventing an immune response by a mammal previously exposed to a reovirus subtype. Recombinant reoviruses may also exhibit different biological activities (e.g., replication activities in neoplastic cells and biodistribution) compared to the original reoviruses. Recombination/reassortment of reovirus genomic segments may occur in nature following infection of a host organism with at least two genetically distinct reoviruses. Recombinant virions can also be generated in cell culture, for example, by co-infection of permissive host cells with genetically distinct reoviruses (Nibert et al., 1996).

Accordingly, the invention contemplates the use of recombinant reoviruses resulting from reassortment of genome segments from two or more genetically distinct reoviruses, including but not limited to, human reovirus, such as type 1 (e.g., strain Lang), type 2 (e.g., strain Jones), and type 3 (e.g., strain Dearing or strain Abney), non-human mammalian reoviruses, or avian reovirus. The invention further contemplates recombinant reoviruses resulting from reassortment of genome segments from two or more genetically distinct reoviruses wherein at least one parental virus is genetically engineered, comprises one or more chemically synthesized genomic segment, has been treated with chemical or physical mutagens, or is itself the result of a recombination event. The invention further contemplates the recombinant reovirus that has undergone recombination in the presence of chemical mutagens, including but not limited to dimethyl sulfate and ethidium bromide, or physical mutagens, including but not limited to ultraviolet light and other forms of radiation.

The invention further contemplates recombinant reoviruses that comprise deletions or duplications in one or more genome segments, that comprise additional genetic information as a result of recombination with a host cell genome, or that comprise synthetic genes.

The virus may be modified by incorporation of mutated coat proteins, such as for example reovirus σ1, into the virion outer capsid. The proteins may be mutated by replacement, insertion or deletion. Replacement includes the insertion of different amino acids in place of the native amino acids. Insertions include the insertion of additional amino acid residues into the protein at one or more locations. Deletions include deletions of one or more amino acid residues in the protein. Such mutations may be generated by methods known in the art. For example, oligonucleotide site directed mutagenesis of the gene encoding for one of the coat proteins could result in the generation of the desired mutant coat protein. Expression of the mutated protein in reovirus infected mammalian cells *in vitro* such as COS1 cells will result in the incorporation of the mutated protein into the reovirus virion particle (Turner and Duncan, (1992); Duncan et al., (1991); Mah et al., (1990).

The virus is preferably a virus modified to reduce or eliminate an immune reaction to the virus ("immunoprotected virus"). Such modifications could include packaging of the virus in a liposome, a micelle or other vehicle to mask the virus from the mammals immune system. Alternatively, the outer capsid of the virus virion particle may be removed since the proteins present in the outer capsid are the major determinant of the host humoral and cellular responses.

In addition to reovirus, other oncolytic viruses may be used to reduce pain. A few such oncolytic viruses are discussed below, and a person of ordinary skill in the art can practice the present invention using additional oncolytic viruses as well according to the disclosure herein and knowledge available in the art. The oncolytic virus may be a member in the family of, for example, myoviridae, siphoviridae, podpviridae, teciviridae, corticoviridae, plasmaviridae, lipothrixviridae, fuselloviridae, poxviridae, iridoviridae, phycodnaviridae, baculoviridae, herpesviridae, adnoviridae, papovaviridae, polydnaviridae, inoviridae, microviridae, geminiviridae, circoviridae, parvoviridae, hepadnaviridae, retroviridae, cyctoviridae, reoviridae, birnaviridae, paramyxoviridae, rhabdoviridae, filoviridae, orthomyxoviridae, bunyaviridae, arenaviridae, leviviridae, picornaviridae, sequiviridae, comoviridae, potyviridae, caliciviridae, astroviridae, nodaviridae, tetraviridae, tombusviridae, coronaviridae, glaviviridae, togaviridae, or barnaviridae. As with reovirus, immunoprotected or reassortant viruses of other oncolytic viruses are also encompassed in the present invention. Furthermore, a combination of at least two oncolytic viruses, including reovirus, can also be employed to reduce pain according to the present inventionl.

Normally, when virus enters a cell, double stranded RNA Kinase (PKR) is activated and blocks protein synthesis, and the virus can not replicate in this cell. Some viruses have developed a system to inhibit PKR and facilitate viral protein synthesis as well as viral replication. For example, adenovirus makes a large amount of a small RNA, VA1 RNA. VA1 RNA has extensive secondary structures and binds to PKR in competition with the double stranded RNA (dsRNA) which normally activates PKR. Since it requires a minimum length of dsRNA to activate PKR, VA1 RNA does not activate PKR. Instead, it sequesters PKR by virtue of its large amount. Consequently, protein synthesis is not blocked and adenovirus can replicate in the cell.

Ras-activated neoplastic cells are not subject to protein synthesis inhibition by PKR, because ras inactivates PKR. These cells are therefore susceptible to viral infection even if the virus does not have a PKR inhibitory system. Accordingly, if the PKR inhibitors in adenovirus, vaccinia virus, herpex simplex virus or parapoxvirus orf virus is mutated so as not to block PKR function anymore, the resulting viruses do not infect normal cells due to protein synthesis inhibition by PKR, but they replicate in ras-activated neoplastic cells which lack PKR activities.

Accordingly, a virus that is modified or mutated such that it does not inhibit PKR function selectively replicates in ras-activated neoplastic cells while normal cells are resistant. Preferably, the virus is an adenovirus mutated in the VA1 region, a vaccinia virus mutated in the K3L and/or E3L region, a herpes simplex virus mutated in the _{γ1}34.5 gene, a parapoxvirus orf virus mutated in the OV20.0L gene, or an influenza virus mutated in the NS-1 gene.

The viruses can be modified or mutated according to the known structure-function relationship of the viral PKR inhibitors. For example, since the amino terminal region of E3 protein interacts with the carboxy-terminal region domain of PKR, deletion or point mutation of this domain prevents anti-PKR function (Chang et al., 1992, 1993, 1995; Sharp et al., 1998; Romano et al., 1998). The K3L gene of vaccinia virus encodes pK3, a pseudosubstrate of PKR. There is a loss-of-function mutation within K3L. Truncations or placing point mutations within the C-terminal portion of K3L protein that is homologous to residues 79 to 83 in eIF-2α abolish PKR inhibitory activity (Kawagishi-Kobayashi et al., 1997).

The oncolytic virus may selectively kill neoplastic cells by carrying a tumor suppressor gene. For example, a virus which expresses the wild type p53 gene can selectively kill the neoplastic cells which become neoplastic due to inactivation of the p53 gene product. Such a virus has been constructed and shown to induce apoptosis in cancer cells that express mutant p53 (Blagosklonny et al., 1996).

A similar approach involves viral inhibitors of tumor suppressors. For example, if the E1B region encoding a 55 kd p53 inhibitor is deleted, as in the ONYX-015 virus (WO 94/18992), the inhibitor is no longer present. As a result, when ONYX-015 enters a normal cell, p53 functions to suppress cell proliferation as well as viral replication, which relies on the cellular proliferative machinery. Therefore, ONYX-015 does not replicate in normal cells. On the other hand, in neoplastic cells with disrupted p53 function, ONYX-015 can replicate and eventually cause the cell to die.

Another example is the Delta24 virus which is a mutant adenovirus carrying a 24 base pair deletion in the EIA region (Fueyo et al., 2000). This region is responsible for binding to the cellular tumor suppressor Rb and inhibiting Rb function, thereby allowing the cellular proliferative machinery, and hence virus replication, to proceed in an uncontrolled fashion. Delta24 has a deletion in the Rb binding region and does not bind to Rb. Therefore, replication of the mutant virus is inhibited by Rb in a normal cell. However, if Rb is inactivated and the cell becomes neoplastic, Delta24 is no longer inhibited. Instead, the mutant virus replicates efficiently and lyses the Rb-deficient cell.

In addition, vesicular stomatitis virus (VSV) selectively kills neoplastic cells (and interferon can be optionally added). A herpes simplex virus 1 (HSV-1) mutant which is defective in ribonucleotide reductase expression, hrR3, was shown to replicate in colon carcinoma cells but not normal liver cells (Yoon et al., 2000). Newcastle disease virus (NDV) replicates preferentially in malignant cells, and the most commonly used strain is 73-T (Reichard et al., 1992; Zorn et al, 1994; Bar-Eli et al, 1996). Vaccinia virus propagated in several malignant tumor cell lines. Encephalitis virus was shown to have an oncolytic effect in a mouse sarcoma tumor, but attenuation may be required to reduce its infectivity in normal cells. Tumor regression have been described in tumor patients infected with herpes zoster, hepatitis virus, influenza, varicella, and measles virus (for a review, see Nemunaitis, 1999).

The oncolytic virus may be naturally occurring or modified. The virus may be chemically or biochemically pretreated (e.g., by treatment with a protease, such as chymotrypsin or trypsin) prior to administration to the neoplastic cells. Pretreatment with a protease removes the outer coat or capsid of the virus and may increase the infectivity of the virus. The virus may be coated in a liposome or micelle (Chandron and Nibert, 1998) to reduce or prevent an immune response from a mammal which has developed immunity to the virus. For example, the virion may be treated with chymotrypsin in the presence of micelle forming concentrations of alkyl sulfate detergents to generate a new infectious subvirion particle.

Pain that is particularly susceptible to reduction by the methods of the invention is associated with solid cancer, for example, sarcoma, melanoma, breast cancer, central nervous system cancer (e.g., neuroblastoma and glioblastoma), peripheral nervous system cancer, lung cancer, prostate cancer, colorectal cancer, thyroid cancer, renal cancer, adrenal cancer, and liver cancer. In particular, the cancer types that are highly associated (e.g., at least about 50%) with pain in Table 1 are contemplated to be treated using the methods of this invention.

The route by which the virus is administered, as well as the formulation, carrier or vehicle, will depend on the location as well as the type of the neoplasm. A wide variety of administration routes can be employed. For example, for a solid neoplasm that is accessible, the virus can be administered by injection directly to the neoplasm. For a hematopoietic neoplasm, for example, the virus can be administered intravenously or intravascularly. For neoplasms that are not easily accessible within the body, such as metastases or brain tumors, the virus is administered in a manner such that it can be transported systemically through the body of the mammal and thereby reach the neoplasm (e.g., intrathecally, intravenously or intramuscularIy). Alternatively, the virus can be administered directly to a single solid neoplasm, where it then is carried systemically through the body to metastases. The virus can also be administered subcutaneously, intraperitoneally, topically (e.g., for melanoma), orally (e.g., for oral or esophageal neoplasm), rectally (e.g., for colorectal neoplasm), vaginally (e.g., for cervical or vaginal neoplasm), nasally or by inhalation spray (e.g., for lung neoplasm).

The virus can be administered systemically, especially to mammals which are immune compromised or which have not developed immunity to the virus. In such cases, the virus administered systemically, i.e. by intravenous injection, will contact the neoplastic cells resulting in lysis of the cells.

Immunocompetent mammals previously exposed to a virus subtype may have developed humoral and/or cellular immunity to that virus subtype. When the virus is administered systemically to immunocompetent mammals, the mammals may produce an immune response to the virus. Such an immune response may be avoided if the virus is of a subtype to which the mammal has not developed immunity, or the virus has been modified as previously described herein such that it is immunoprotected, for example, by protease digestion of the outer capsid or packaging in a micelle.

Alternatively, it is contemplated that the immunocompetency of the mammal against the virus may be suppressed either by the co-administration of pharmaceuticals known in the art to suppress the immune system in general (Cuff et al., (1998) or alternatively by administration of anti-antivirus antibodies. The humoral immunity of the mammal against virus may also be temporarily reduced or suppressed by plasmaphoresis of the blood to remove the anti-virus antibodies. The humoral immunity of the mammal against virus may additionally be temporarily reduced or suppressed by intravenous administration of non-specific immunoglobulin to the mammal.

It is contemplated that the virus may be administered to immunocompetent mammals immunized against the virus in conjunction with the administration of anti-antivirus antibodies. Anti-antivirus antibodies used in this invention are selected, for example, from anti-antireovirus antibodies, anti-antiadenovirus antibodies, anti-antiHSV antibodies, anti-antivaccinia virus antibodies, anti-antiinfluenza antibodies and anti-antiparapoxvirus orf virus antibodies. Such antibodies can be made by methods known in the art (see, for example, Harlow et al.,1988). Such anti-antivirus antibodies may be administered prior to, at the same time or shortly after the administration of the virus. Preferably an effective amount of the anti-antivirus antibodies are administered in sufficient time to reduce or eliminate an immune response by the mammal to the administered virus.

In one embodiment of this invention a course of virus therapy is administered one or more times. Following the first administration of virus therapy particular immune constituents that may interfere with subsequent administrations of virus are removed from the patient. These immune constituents include B cells, T cells, antibodies, and the like.

Removal' of either the B cell or T cell population can be accomplished by several methods. In one method, the blood may be filtered and heme-dialysis may be performed. Another method is the filtration of the blood coupled with extra corporeal compounds that can remove the cell populations, for example, with immobilized antibodies that recognize specific receptors on the cell population which is to be remove. Yet another method for removal of a cell population is by immune suppression. This can be done by first line radiation therapy or by cyclic steroids such as cyclosporin.

Selective removal of anti-virus antibodies can also prevent the patient's immune system from removing therapeutically administered virus. Preventing antibody interaction with the administered virus may also assist systemic treatment strategies. Antibodies can be removed by several methods, including heme-dialysis and passing the blood over immobilized virus (selective antibody removal); by removal of all IgG antibodies by heme-dialysis and passing the blood over immobilized protein A (commercially available as PROSORBA, Cypress Bioscience, San Diego, CA); or by administration of humanized anti-idiotypic antibodies, where the idiotype is against the virus to be administered.

Another method of this invention is to allow virus to act systemically without impairing normal immune function by masking or impairing immune recognition of virus. To prevent the patient's immune system from recognizing the administered virus, the virus may be coated with non-virotoxic humanized antibodies, such as coating with the F_{ab} portion of the antibody, or coated in a micelle.

Additionally, the virus may be treated with chymotrypsin to yield an infectious subviral particle (ISVP). An ISVP may be used either alone or in combination with whole virus to provide an agent that is either poorly recognized has not been previously prevented by the patient's immune system.

Another embodiment of this invention includes the removal of virus from the patient following administration. Since this method may be used on patients that are either immune suppressed or immune incompetent, it may be of importance to remove virus from the blood stream following the course of treatment. The virus may be removed by affinity chromatography using extra corporeal anti-virus antibodies associated with heme dialysis, B-cell proliferative agents, or adjuvants to stimulate immune response against the virus such as UV inactivated virus or Freund's adjuvant.

### Compositions and Kits

This invention also includes pharmaceutical compositions which contain, as the active ingredients, one or more of the oncolytic viruses and at least one analgesic. Alternatively, the pharmaceutical composition may comprise two components, each of which is a pharmaceutical composition comprising an oncolytic virus and an analgesic, respectively. The two components can be administered together or separately.

The pharmaceutical composition may also comprise pharmaceutically acceptable carriers or excipients. In making the compositions of this invention, the active ingredients are usually mixed with an excipient, diluted by an excipient or enclosed within such a carrier which can be in the form of a capsule, sachet, paper or other container. When the pharmaceutically acceptable excipient serves as a diluent, it can be a solid, semi-solid, or liquid material, which acts as a vehicle, carrier or medium for the active ingredients. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredients after administration to the patient by employing procedures known in the art.

For preparing solid compositions such as tablets, the principal active ingredients are mixed with a pharmaceutical excipient to form a solid preformulation composition containing a homogeneous mixture of a compound of the present invention. When referring to these preformulation compositions as homogeneous, it is meant that the active ingredients are dispersed evenly throughout the composition so that the composition may be readily subdivided into equally effective unit dosage forms such as tablets, pills and capsules.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described herein. Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner.

Another preferred formulation employed in the methods of the present invention employs transdermal delivery devices ("patches"). Such transdermal patches may be used to provide continuous or discontinuous infusion of the virus in controlled amounts. The construction and use of transdermal patches for the delivery of pharmaceutical agents is well known in the art. See, for example, U.S. Patent 5,023,252, herein incorporated by reference. Such patches may be constructed for continuous, pulsatile, or on demand delivery of pharmaceutical agents.

Other suitable formulations for use in the present invention can be found in *Remington's Pharmaceutical Sciences.*

The virus or the pharmaceutical composition comprising the virus may be packaged into convenient kits providing the necessary materials packaged into suitable containers. It is contemplated the kits may also include chemotherapeutic agents and/or anti-antivirus antibody.

The virus is administered in an amount that is sufficient to reduce pain (e.g., an "effective amount"). Lessening of pain may occur with or without actual tumor regression. The reduction of pain may be concomitant with reduction in size of the neoplasm, or elimination of the neoplasm. The reduction in size of the neoplasm, or in a complete elimination of the neoplasm, is generally caused by lysis of neoplastic cells ("oncolysis") by the virus (U.S. Patents 6,110,461; 6,136,307; 6,261,555; and 6,344,195).

Preferably the effective amount is that amount able to completely suppress pain. Preferably the effective amount is from about 1 plaque forming units (pfu) to about 10¹⁵ pfu, more preferably from about 10² pfu to about 10¹³ pfu. For example, for treatment of a human, approximately 10² to 10¹⁷ pfu of reovirus can be used, depending on the type, size and number of tumors present. The effective amount will be determined on an individual basis and may be based, at least in part, on consideration of the type of virus; the chosen route of administration; the individual's size, age, gender; the severity of the patient's symptoms; the size and other characteristics of the neoplasm; and the like. The course of therapy may last from several days to several months or until diminution of pain, and/or the disease, is achieved.

The virus can be administered in a single dose, or multiple doses (i.e., more than one dose). The multiple doses can be administered concurrently, or consecutively (e.g., over a period of days or weeks). The virus can also be administered to more than one neoplasm in the same individual. The compositions are preferably formulated in a unit dosage form, each dosage containing from about 10² pfu to about 10¹³ pfu of the virus.

It is contemplated that the virus may be administered in conjunction with or in addition to opiod therapy or in addition to administering non-opioid and adjuvant analgesics. It is also contemplated that the virus may be administered in conjunction with or in addition to hormonal therapy or immunotherapy.

It is further contemplated that the virus of the present invention may be administered in conjunction with or in addition to radiation therapy. Radiotherapy, also called radiation therapy, is the treatment of cancer and other diseases with radiation, typically ionizing radiation. The irradiating agent can be any irradiating agent known in the art, including but not limited to X-rays, gamma rays (e.g., gamma rays produced by radium, uranium, or cobalt 60), and particle beam (e.g., electrons, neutrons, pions, and heavy ions). The irradiation may be in the form of external radiotherapy or internal radiotherapy (including brachytherapy, interstitial irradiation, and intracavitary irradiation). The irradiating agents may be linked to an antibody, as in radioimmunotherapy, or employed during a surgery, as in intraoperative radiotherapy.

It is yet further contemplated that the virus of the present invention may be administered in conjunction with or in addition to known anticancer compounds or chemotherapeutic agents. Chemotherapeutic agents are compounds which may inhibit the growth of tumors. Such agents, include, but are not limited to, 5-fluorouracil, mitomycin C, methotrexate, hydroxyurea, cyclophosphamide, dacarbazine, mitoxantrone, anthracyclins (Epirubicin and Doxurubicin), antibodies to receptors, such as herceptin, etopside, pregnasome, platinum compounds such as carboplatin and cisplatin, taxanes such as taxol and taxotere, hormone therapies such as tamoxifen and anti-estrogens, interferons, aromatase inhibitors, progestational agents and LHRH analogs.

The following examples are offered to illustrate this invention and are not to be construed in any way as limiting the scope of the present invention.

### EXAMPLES

In this application, the following abbreviations have the following meanings. Abbreviations not defined have their generally accepted meanings.
- °C =: degree Celsius
- hr =: hour
- min =: minute
- µM =: micromolar
- mM =: millimolar
- M =: molar
- ml =: milliliter
- µl =: microliter
- mg =: milligram
- µg =: microgram
- PBS =: phosphate buffered saline
- EGF =: epidermal growth factor
- PDGF =: platelet derived growth factor
- PKR =: double stranded RNA kinase

### EXAMPLE 1

### Phase I Clinical Study:

### Use of Reovirus for Reducing Pain Associated with Malignant Melanoma

A 54-year old female patient was suffering from malignant melanoma, which formed multiple lesions (more than 10) over the whole body and failed to respond to conventional anticancer treatments. The patient was permanently on narcotics due to pain associated with the lesion of the left posterior neck.

The patient received three intratumoral injections of 10⁹ pfu of the Dearing strain of reovirus serotype 3 into the lesion on the left posterior neck. The Dearing strain of reovirus serotype 3 used in these studies was obtained by a method disclosed in U.S. Patent Application Publication No. 20020037576.

Surprisingly, one week following injection, the patient reported diminished pain at the treatment site and was taken off narcotics. There was no pain at the treatment site during a 8-10 week period after the injection. As shown by a punch biopsy conducted from the left shoulder after two weeks following the injection, there were no significant side effects or adverse affects related to the administration of the virus in the patient. Histological results showed only some dermal fibrosis and mild perivascular chronic inflammation.

### EXAMPLE 2

### Phase I Clinical Study:

### Use of Reovirus for Reducing Pain Associated with Ewing's Sarcoma

A 27-year old male terminal patient with Ewing's sarcoma received a single intratumoral injection of 10⁹ pfu of the Dearing strain of reovirus serotype 3 into a sacroiliac site. The Dearing strain of reovirus serotype 3 used in these studies was obtained by a method disclosed in U.S. Patent Application Publication No. 20020037576. One week following injection, the patient reported diminished pain at the injection site. There was no pain at the treatment site during the next several weeks after the injection.

All of the above publications, patent applications and patents are herein incorporated by reference in their entirety to the same extent as if each individual publication, patent application or patent was specifically and individually indicated to be incorporated by reference in its entirety.

While this invention has been particularly shown and described with references to preferred embodiments thereof, it will be understood by those skilled in the art that various changes in form and details may be made therein without departing from the spirit and scope of the invention as defined by the appended claims.

## Claims

1. Use of an effective amount of at least one oncolytic virus for the preparation of a pharmaceutical composition for reducing pain in a mammal suffering from a neoplasm.

2. The use of claim 1 wherein at least one analgesic in an amount that is less than the amount of the analgesic required to reduce pain in the absence of the oncolytic virus in the mammal is further to be administered.

3. The use of claim 2 wherein the analgesic is selected from the group consisting of opioid analgesics, non-opioid analgesics, codeine, fentanyl, hydromorphone, levorphanol, meperidine, methadone, morphine, oxycodone, oxymorphone, propoxyphene, buprenorphine, butorphanol, dezocine, nalbuphine, and pentazocine.

4. The use of any one of claims 1 to 3, wherein the neoplasm is a solid neoplasm, is metastatic or is terminal.

5. The use of claim 4, wherein the oncolytic virus is to be administered by injection into or near the solid neoplasm.

6. The use of any one of claims 1 to 5, wherein more than one type of the oncolytic virus or more than one strain of the oncolytic virus are to be administered.

7. The use of any one of claims 1 to 6, wherein the oncolytic virus is to be administered in a single dose or in more than one dose.

8. The use of any one of claims 1 to 7, wherein the oncolytic virus is a reovirus.

9. The use of claim 8, wherein the reovirus is selected from the group consisting of mammalian reoviruses and avian reoviruses.

10. The use of claim 9, wherein the reovirus is a human reovirus.

11. The use of any one of claims 8 to 10, wherein the reovirus is selected from the group consisting of serotype 1 reovirus, serotype 2 reovirus and serotype 3 reovirus.

12. The use of any one of claims 8 to 11, wherein the reovirus is a recombinant reovirus.

13. The use of any one of claims 8 to 12, wherein approximately 1 to 10¹⁵ plaque forming units of reovirus are to be administered.

14. The use of any one of claims 1 to 13, wherein the mammal is a human.

15. The use of any one of claims 1 to 14 wherein an effective amount of a chemotherapeutic agent is further to be administered.

16. The use of any one of claims 1 to 14 wherein the oncolytic virus is to be administered in conjunction with radiation therapy, in conjunction with hormonal therapy or in conjunction with immunotherapy.

17. A pharmaceutical composition comprising two components as follows:
(a) a first component which is a first pharmaceutical composition comprising an effective amount of an oncolytic virus as defined in any one of claims 1 or 6 to 12; and
(b) a second component which is a second pharmaceutical composition comprising an effective amount of an analgesic.

18. The pharmaceutical composition of claim 17 wherein the analgesic is selected from the group consisting of opioid analgesics and non-opioid analgesics.
